# EUROPEAN PATENT APPLICATION

(11) **EP 4 140 522 A1**
(43) Date of publication of application: **01.03.2023**
(21) Application number: 21826004.0
(22) Date of filing: 13.05.2021
(51) Int. Cl.: A61M 5/32, A61M 5/178, A61M 5/31

(54) **AUTOMATICALLY TRIGGERED NEEDLE PROTECTION DEVICE AND INSULIN PEN NEEDLE**

(30) Priority: 18.06.2020 CN 202010559508
(71) Applicant: Promisemed Hangzhou Meditech Co., Ltd., Hangzhou, Zhejiang 311121 (CN)
(72) Inventor: CHAOYU, Hu, Hangzhou, Zhejiang 311121 (CN)
(74) Representative: Cabinet Chaillot
(86) International application number: PCT/CN2021/093643
(87) International publication number: WO 2021/254050

(57) **Abstract**

The present invention relates to the technical field of medical instruments, in particular to an automatically-triggered needle protector and an insulin pen needle. The automatically-triggered needle protector at least includes a needle hub, a protective sleeve, a shield, a spring and a support part. The support part at least includes an initial state and a support state and is configured to be switched into the support state from the initial state. In the initial state, a support base body of the support part is in contact with an upper end of the needle hub, and a circumferential limiting mechanism is arranged between the support base body and a needle body installation portion; and in the support state, the support part disengages from sliding grooves, and lower ends of support arms are located above support surfaces. In the above structure, the shield is automatically triggered, a secondary operation is not needed in a protection process, and operation is simple, convenient and fast; and the support part is located on an inner side and supported in a space between the shield and the needle hub in an axial direction, support safety and reliability are better, and meanwhile, a protection state of a needle is not likely to be damaged from outside, thereby effectively preventing secondary usage of the insulin pen needle.

## Description

### Technical Field

The present invention relates to the technical field of medical instruments, in particular to an automatically-triggered needle protector and an insulin pen needle.

### Background

In the prior art, there are many types of needle protectors for insulin pen needles, mainly including a manual protection type and an automatic protection type. For the insulin pen needle in the manual protection type, after injection is finished, a needle protective cover needs to be manually pushed to a protection position, not facilitating operation.

For the existing insulin pen needle in the automatic protection type, a needle protective cover will be triggered in the injection process so as to automatically cover the syringe needle after the injection is finished. Currently, trigger mechanisms in the automatic protection type are commonly achieved through cooperation of track grooves and protrusions between needle protective covers and shells. The automatic trigger form has the defects that a structure is complex, and after a trigger, reliability is poor, and damage is likely to happen.

### SUMMARY

The present invention aims to solve a technical problem by providing a structure-improved automatically-triggered needle protector and an insulin pen needle.

To solve the above technical problem, a technical solution is provided by the present invention as below: the automatically-triggered needle protector at least includes;
a needle hub provided with a syringe needle, where at least one first groove wall downwards extending from an upper end of the needle hub is arranged on an outer side of the needle hub in a circumferential direction, and support surfaces extending from top ends of the first groove walls in a direction away from the first groove walls are arranged at the upper end of the needle hub;
a protective sleeve sleeved an outer side of the needle hub and fixedly connected to the needle hub, where a first cavity with an upper end open is formed between the protective sleeve and the needle hub;
a shield installed in the first cavity, where the shield has an extension state and a retraction state in the first cavity and is configured to be movable between the extension state and the retraction state, an inner side of the shield is provided with second groove walls opposite to the first groove walls, a sliding groove is formed between each first groove wall and the corresponding second groove wall, and a lower end of each second groove wall is provided with a hook extending towards one side of the corresponding first groove wall;
a spring installed in the first cavity and located between the shield and a cavity bottom of the first cavity; and
a support part, where the support part at least includes a support base body and support arms corresponding to the sliding grooves, the sides, facing the second groove walls, of the support arms are provided with elastic arms, upper ends of the elastic arms are located at positions, close to upper ends, of the support arms, a first gap is arranged between a lower end of each elastic arm and the corresponding support arm, and a maximum width formed by the elastic arms and the support arms is larger than a width of the sliding grooves; and
the support part at least includes an initial state and a support state and is configured to be switched into the support state from the initial state, where in the initial state, the support base body of the support part is in contact with the upper end of the needle hub; and in the support state, the support part disengages from the sliding grooves, and lower ends of the support arms are located above the support surfaces.

In a preferable embodiment, two sliding grooves are evenly distributed in a circumferential direction.

In a preferable embodiment, a needle body installation portion is arranged at an upper end of a needle hub, a support base body of a support part is provided with an installation hole adaptive to the needle body installation portion, a circumferential limiting mechanism is arranged between the needle body installation portion and the support base body and includes at least one limiting protrusion arranged on an outer side of the needle body installation portion and limiting grooves formed in an inner side of the installation hole and adaptive to the limiting protrusions.

In a preferable embodiment, the sides, close to the first groove walls, of support surfaces are required not to be lower than the sides away from the first groove walls.

In a preferable embodiment, a first axial guide mechanism and an axial anti-disengaging limiting mechanism are arranged between a shield and a protective sleeve.

In a preferable embodiment, an axial anti-disengaging limiting mechanism includes a first limiting step arranged at an upper-end open position of a protective sleeve and a second limiting step arranged on an outer side of a lower end of a shield.

In a preferable embodiment, an upper end of a needle hub is provided with limiting convex portions with upper ends protruding out of support surfaces, one side of each limiting convex portion is provided with a first limiting surface opposite to a corresponding first groove wall, and the other side of each limiting convex portion is provided with a second limiting surface connected to the end, away from the corresponding first groove wall, of the corresponding support surface.

The insulin pen needle at least includes an automatically-triggered needle protector and an initial protective cover an outer side of the automatically-triggered needle protector.

In a preferable embodiment, a second axial guide mechanism is arranged between a protective sleeve and an initial protective cover.

In a preferable embodiment, a shield is provided with a top wall, the top wall is provided with a needle via hole for containing a syringe needle to pass through and further provided at least one through groove penetrating through the top wall, limiting rods corresponding to the through grooves and configured into an initial state are arranged in the initial protective cover, and the limiting rods penetrate through the through grooves and abut against an upper surface of a support base body.

According to the insulin pen needle in the embodiment, a working principle of the automatically-triggered needle protector includes: in the initial state, the support part is installed on the needle hub, the shield covers the syringe needle, one side of each support arm of the support part abuts against the first groove wall of the corresponding sliding groove, in the process of insulin injection, the shield axially moves, the spring is compressed, in the process of axial movement of the hooks of the second groove walls, the hooks firstly move to make contact with outer sides of the elastic arms and then continue to axially move, each elastic arm is extruded towards one side of the corresponding support arm till upper surfaces of the hooks move to positions below the elastic arms, the elastic arms reset to the sides away from the support arms at the time and make contact with the second groove walls, lower ends of the elastic arms are located above the hooks, and in the state, the elastic arms are still in an elastic energy storage state.

After the injection is finished, the shield axially moves to cover the syringe needle under the reset action of the spring, in the process, the hooks act on the lower ends of the elastic arms to drive the support body to axially move till the lower ends of the support arms disengage from the first groove walls, and stored elastic energy of the elastic arms is further released at the time to drive the support part to rotate to the lower ends of the support arms and above the support surfaces.

In the state, since the maximum width formed by the elastic arms and the support arms is larger than the width of the sliding grooves, the elastic arms and the support arms cannot enter the sliding grooves again, so that the support part is made to be supported in a space between the shield and the needle hub, then, the shield is pressed again, and a needle tip cannot be exposed again based on a supporting function of the support part.

Compared with the prior art, the automatically-triggered protector for the insulin pen needle has following beneficial effects:
(1) before and after injection, the syringe needle is located in the shield, an automatic trigger is finished in the injection process, a secondary operation is not needed in a protection process, and the operation is simple, convenient, and fast;
(2) the support part is located on an inner side and supported in the space between the shield and the needle hub in the axial direction, support safety and reliability are better, and meanwhile, a protection state of a needle is not likely to be damaged from outside, thereby effectively preventing secondary usage of the insulin pen needle; and
(3) the elastic arms have multiple functions in a trigger process, and firstly, a cooperative relationship is generated between the hooks and the support body, and accordingly the support part is driven to synchronously move due to movement of the shield; secondly, when the support part moves till the support arms disengage from the first groove walls, elastic potential energy stored by the elastic arms drives the support part to rotate to enter a support state; and thirdly, in the support state, since the maximum width formed by the elastic arms and the support arm is larger than the width of the sliding grooves, the elastic arms and the support arms cannot integrally enter the sliding grooves again. The elastic arms have the technical advantages of being simple in structure and ingenious in concept.

### BRIEF DESCRIPTION OF THE DRAWINGS

FIG. 1 is an exterior structure schematic diagram of an insulin pen needle in the embodiment;
FIG. 2 is an explosion state structural schematic diagram of the insulin pen needle in the embodiment;
FIG. 3 is a structural schematic diagram of an initial protective cover in the embodiment;
FIG. 4 is a structural schematic diagram of a needle hub in the embodiment;
FIG. 5 is a side-view structure schematic diagram of the needle hub in the embodiment;
FIG. 6 is a cooperative structure schematic diagram of the needle hub and a support part in the embodiment;
FIG. 7 is a structural schematic diagram of the support part in the embodiment;
FIG. 8 is an interior structure schematic diagram of a protective sleeve in the embodiment;
FIG. 9 is an exterior structure schematic diagram of the protective sleeve in the embodiment;
FIG. 10 is an another-angle exterior structure schematic diagram of the protective sleeve in the embodiment;
FIG. 11 is an exterior structure schematic diagram of a shield in the embodiment;
FIG. 12 is an another-angle exterior structure schematic diagram of the shield in the embodiment;
FIG. 13 is a local section-view structure schematic diagram of the insulin pen needle in an initial state in the embodiment;
FIG. 14 is an exterior structure schematic diagram of an automatically-triggered needle protector in an initial state in the embodiment;
FIG. 15 is a local section-view structure schematic diagram of the automatically-triggered needle protector shown in FIG. 14;
FIG. 16 is a local section-view structure schematic diagram of the automatically-triggered needle protector in the embodiment in an injection triggering process;
FIG. 17 is a local section-view structure schematic diagram of the automatically-triggered needle protector in an injection state in the embodiment; and
FIG. 18 is a local section-view structure schematic diagram of the automatically-triggered needle protector in the embodiment in a syringe needle protecting state after triggering.

### DETAILED DESCRIPTION

To make purposes, a technical solution and advantages of the present invention more clearly understood, the present invention is further described in detail by combining drawings and embodiments as below. It should be understood that the specific embodiments described herein are only used for explaining the present invention but not used for limiting the present invention.

In the description of the present invention, it needs to be understood that orientation or position relationships indicated by terms "upper", "lower", "front", "rear", "inner", "outer", and the like are based on the drawings, and are merely used for conveniently describing the present invention and simplifying the description, but not indicate or imply that indicated devices or components must be in specific orientations or structured and operated in specific orientations, and thus should not be understood as limits to the present invention.

In the description of the present invention, it needs to be explained that except additional specific regulations and limitations, terms "install", "joint" and "connect" should be generally understood, such as fixed connection, integrated connection and detachable connection; it can also be communication in two components; and it can be direct connection or indirect connection through intermedia. Ordinary skill in the art can understand specific meanings of the above terms in the present invention.

In the embodiment, one end, making contact with a patient, of the insulin pen needle is limited as an upper end, and various components are consistent to the whole insulin pen needle in upper-lower-end direction.

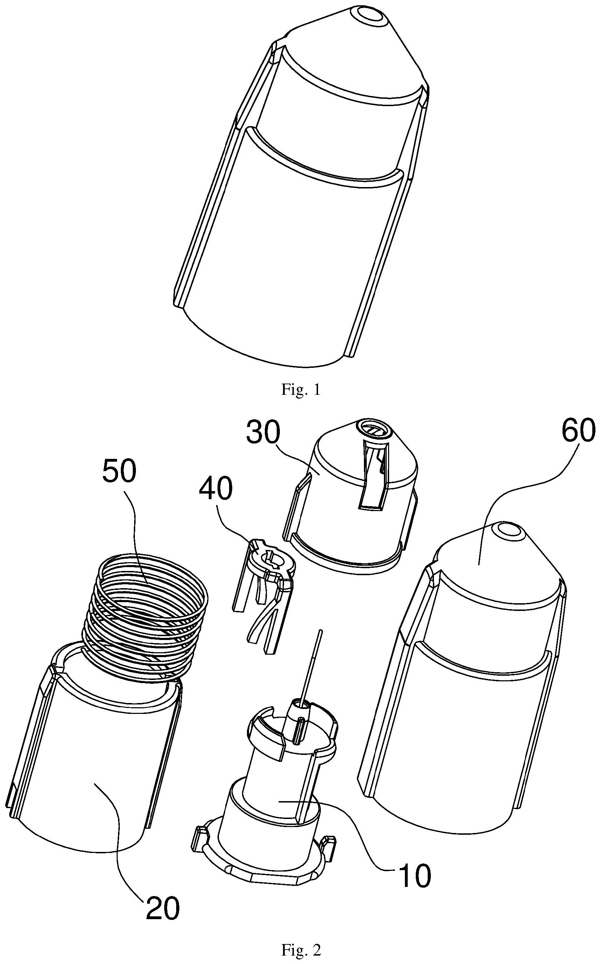

An insulin pen needle in the embodiment, as shown in FIG. 1-2, includes an automatically-triggered needle protector and an initial protective cover 60 covering an outer side of the automatically-triggered needle protector in an initial state.

The automatically-triggered needle protector in the embodiment, as shown in FIG. 2, includes a needle hub 10, a protective sleeve 20, a shield 30, a support part 40 and a spring 50.

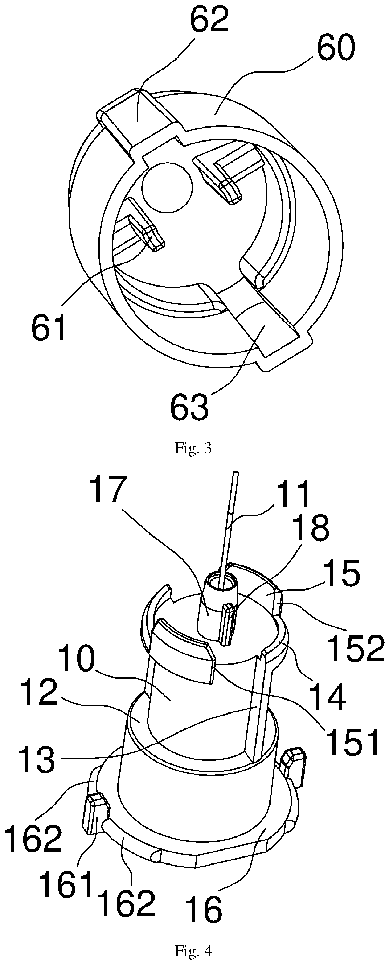

As shown in FIG. 4- 5, an upper end of the needle hub 10 of the embodiment is provided with a needle body installation portion 17 provided with a syringe needle 11. In the embodiment, two evenly-distributed first groove walls 13 are arranged on an outer side of the needle hub 10 in a circumferential direction and downwards extend from an upper end portion of the needle hub. In the embodiment, support surfaces 14 extending from top ends of the first groove walls 13 in a direction away from the first groove walls 13 are arranged at the upper end of the needle hub 10.

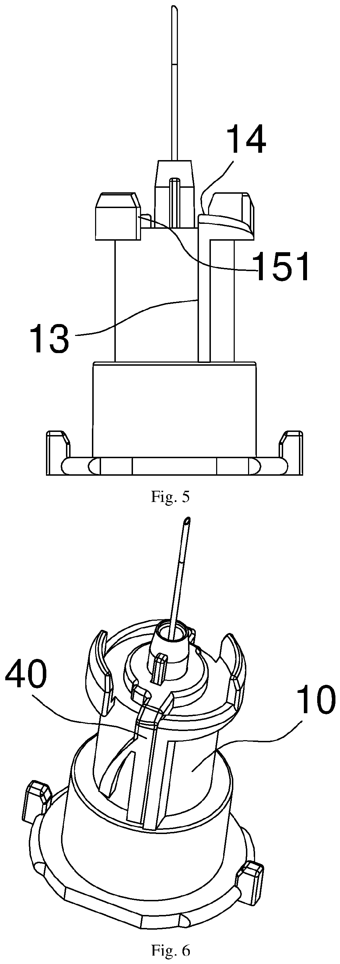

As shown in FIG. 5, in the embodiment, preferably, the support surfaces 14 are gradually lowered from the upper ends of the first groove walls 13 in the direction away from the first groove walls. Of course, the support surfaces may be planes or rough or concave-convex upper surfaces, and it only needs to be met that the ends, close to the first groove walls, of the support surfaces are not lower than the ends away from the first groove walls.

In the embodiment, the upper end of the needle hub 10 is provided with limiting convex portions 15 with upper ends protruding out of the support surfaces 14, one side of each limiting convex portion 15 is provided with a first limiting surface 151 opposite to the corresponding first groove wall 13, and the other side of each limiting convex portion 15 is provided with a second limiting surface 152 connected to the end, away from the corresponding first groove wall 13, of the corresponding support surface 14.

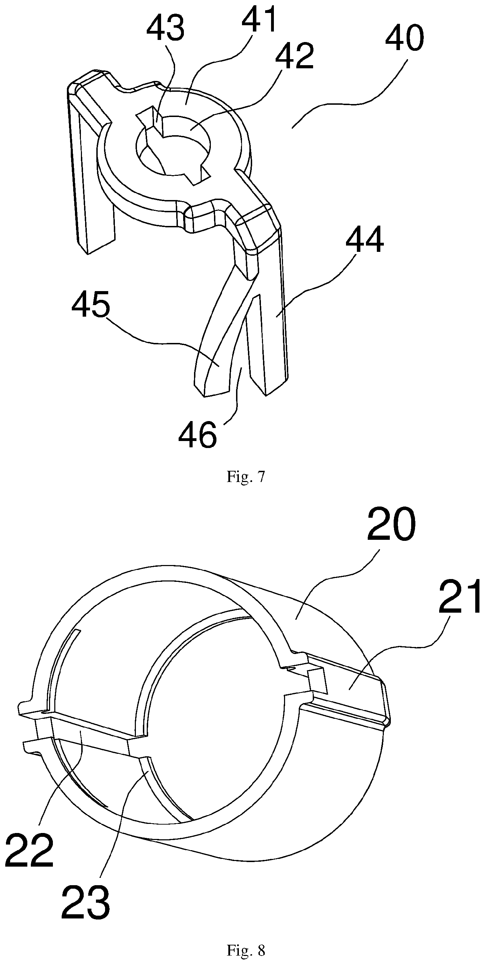

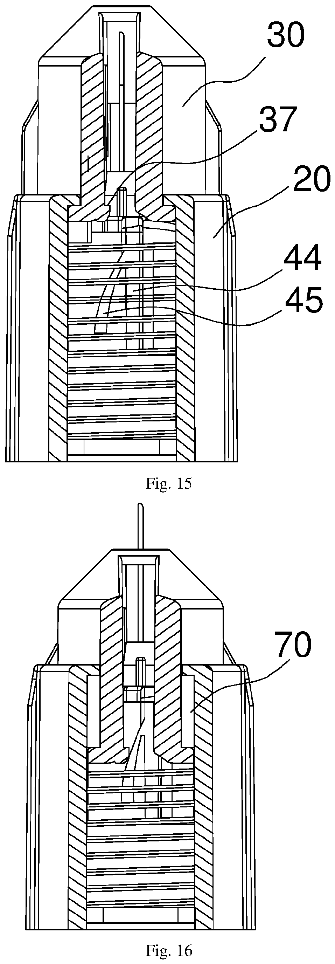

A structure of the protective sleeve 20 in the embodiment is shown in FIG. 8-10, the protective sleeve 20 sleeves the outer side of the needle hub 10, a lower end of the protective sleeve 20 is fixedly connected to a lower end of the needle hub 10, and as shown in FIG. 16, a first cavity 70 with an upper end open is formed between the protective sleeve 20 and the needle hub 10.

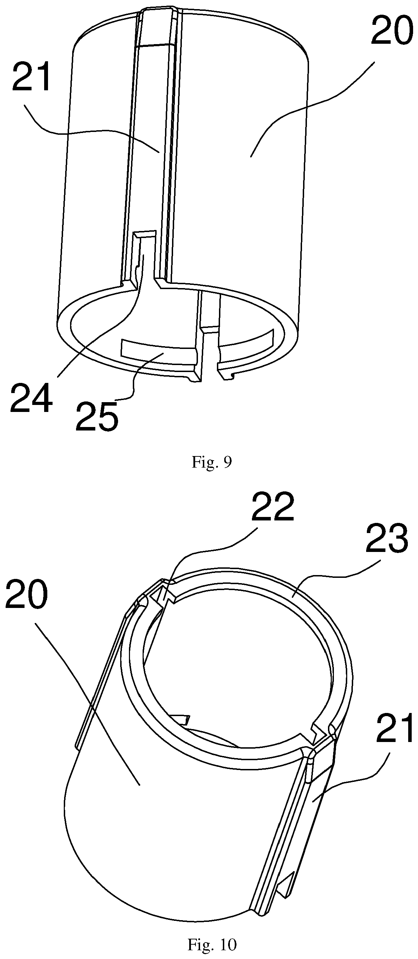

A preferable connection mode between the protective sleeve 20 and the needle hub 10 is shown in FIG. 4, the lower end of the needle hub is provided with a flange 16, two clamping blocks 161 are evenly distributed on an outer edge of the flange 16 in a circumferential direction, and two sides of each clamping block 161 are provided with protruding edges 162. Correspondingly, as shown in FIG. 9, the lower end of the protective sleeve is provided with first clamping grooves 24 adaptively connected to the clamping blocks 161, and a position, close to the lower end, of an inner wall of the protective sleeve is provided with second clamping grooves 25 distributed in two sides of the first clamping grooves 24, and the second clamping grooves 25 are adaptively connected to the protruding edges 162.

In the embodiment, connection between the clamping blocks 161 and the first clamping grooves 24 limits circumferential displacement therebetween, and connection between the protruding edges 162 and the second clamping grooves 25 limits axial displacement therebetween.

Shield 30 in the embodiment is installed in the first cavity 70 and has an extension state and a retraction state, where in the extension state, the shield completely covers the syringe needle to prevent a needle tip from being exposed; and the retraction state is an injection state in which the syringe needle stretches out of the upper end of the shield. In the embodiment, the shield is continuously switched between the extension state and the retraction state.

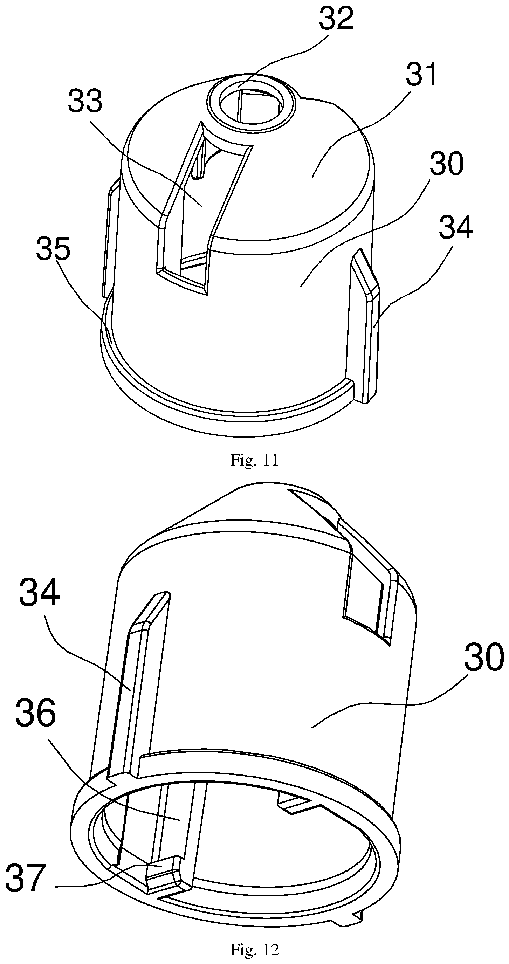

As shown in FIG. 11 and FIG. 12, an inner side of the shield 30 in the embodiment is provided with second groove walls 36 opposite to the first groove walls 13, and the sides, away from the first groove walls, of the second groove walls are in contact fit with the first limiting surfaces 151 to be limited by the first limiting surfaces 151.

In the embodiment, a lower end of each second groove wall 36 is provided with a hook 37 extending towards one side of the corresponding first groove wall. A sliding groove is formed between each first groove wall 13 and the corresponding second groove wall 36 and is characterized in that the first groove walls 13 are fixed groove walls, and the second groove walls 36 can move in an axial direction.

In the embodiment, a first axial guide mechanism and an axial anti-disengaging limiting mechanism are arranged between the shield 30 and the protective sleeve 20. The axial anti-disengaging limiting mechanism, shown in FIG. 8 and FIG. 11, includes a first limiting step 23 arranged at an upper-end open position of the protective sleeve 20 and a second limiting step 35 arranged on an outer side of the lower end of the shield. The protective sleeve and the shield cannot disengage in the axial direction based on the cooperation of the first limiting step 23 and the second limiting step 35.

The first axial guide mechanism in the embodiment, shown in FIG. 8, FIG. 11 and FIG. 12, includes a pair of first guide grooves 22 formed in the protective sleeve 20 and first guide ribs 34 arranged on the outer side of the shield, and the first guide grooves 22 and the first guide ribs 34 are matched to enable the shield to move only in the axial direction.

It needs to be explained that arranging the first axial guide mechanism enables the shield in the embodiment to move only in the axial direction. Of course, it is only a preferable implementation mode but not a unique limitation, and as an optional equal implementation mode, the shield can also be switched between the extension state and the retraction state in a spiral lifting manner.

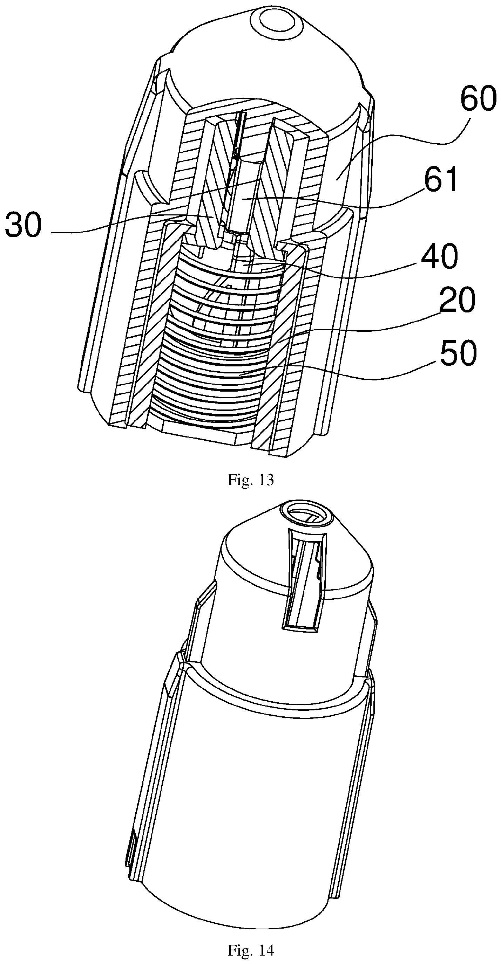

As shown in FIG. 13- 18, in the embodiment, the spring 50 is installed in the first cavity 70 and located between the shield 30 and a cavity bottom (the flange 16) of the first cavity 70.

The support part 40 in the embodiment, shown in FIG. 7, includes a support base body 41 and a pair of support arms 44 corresponding to the sliding grooves, the sides, facing the second groove walls, of the support arms 44 are provided with elastic arms 45, upper ends of the elastic arms 45 are located at positions, close to upper ends, of the support arms 44, and a first gap 46 is arranged between a lower end of each elastic arm 45 and the corresponding support arm 44, where a maximum width formed by the elastic arms and the support arms is larger than a width of the sliding grooves.

Support part 40 in the embodiment includes an initial state and a support state and is configured to be switched into the support state from the initial state, where in the initial state, the support base body of the support part is in contact with the upper end of the needle hub; and in the support state, the support part disengages from the sliding grooves, and lower ends of the support arms are located above the support surfaces.

In the embodiment, as shown in FIG. 6 and FIG. 7, the support base body 41 of the support part 40 is provided with an installation hole 42 adaptive to the needle body installation portion 17.

As an optimization, a circumferential limiting mechanism is arranged between the support part 40 and the needle body installation portion 17 in the initial state. In the embodiment, the circumferential limiting mechanism includes two limiting protrusions 18 arranged on an outer side of the needle body installation portion and limiting grooves 43 which are formed in an inner side of the installation hole 42 and are adaptive to the limiting protrusions 18.

As an optimization, in the embodiment, a second axial guide mechanism is arranged between the initial protective cover 60 and the protective sleeve 20. As shown in FIG. 3 and FIG. 8-10, the second axial guide mechanism includes a pair of second guide grooves 63 formed in an inner wall of the initial protective cover 60 and second guide ribs 21 arranged on an outer side of the protective sleeve 20. As an optimization, the second guide ribs 21 are adaptive to the first guide grooves 22 in position.

As an optimization, in the embodiment, positions, corresponding to the second guide grooves 63, of the outer wall of the initial protective cover 60 are provided with convex ribs 62.

According to the insulin pen needle in the embodiment, the initial state is shown in FIG. 13-15, and in the state, the initial protective cover 60 covers the outer side to prevent the insulin pen needle from being mistakenly triggered.

A preferable implementation mode is shown in FIG. 11, in the embodiment, shield 30 is provided with a top wall 31, the top wall 31 is provided with a needle via hole 32 containing the syringe needle to pass through, and the top wall 31 is further provided with at least two through grooves 33 penetrating through the top wall. Correspondingly, as shown in FIG. 3, limiting rods 61 corresponding to the through grooves 33 are arranged in the initial protective cover 60.

As shown in FIG. 13, in the initial state, the limiting rods 61 penetrate through the through grooves 33 and abut against an upper surface of the support part, thereby effectively limiting axial displacement of the support part and preventing the axial displacement of the support part due to swinging in an unused state.

In the embodiment, the through grooves 33 further have a function of facilitating observation of the state of the syringe needle in the shield.

A working principle of the automatically-triggered needle protector for the insulin pen needle in the embodiment includes:
in the unused state as shown in FIG. 13, the initial protective cover sleeves outer sides of the protective sleeve and the shield, and in the state, the limiting rods penetrate through the through grooves and abut against the upper surface of the support part, thereby effectively limiting the axial displacement of the support part and preventing the axial displacement of the support part due to swinging in the unused state.

During usage, the initial protective cover is taken off firstly in an initial state before injection, as shown in FIG. 13-15, the support part is installed on the needle hub, the shield is in the extension state and covers the syringe needle, and one side of each support arm of the support part abuts against the first groove wall of the corresponding sliding groove.

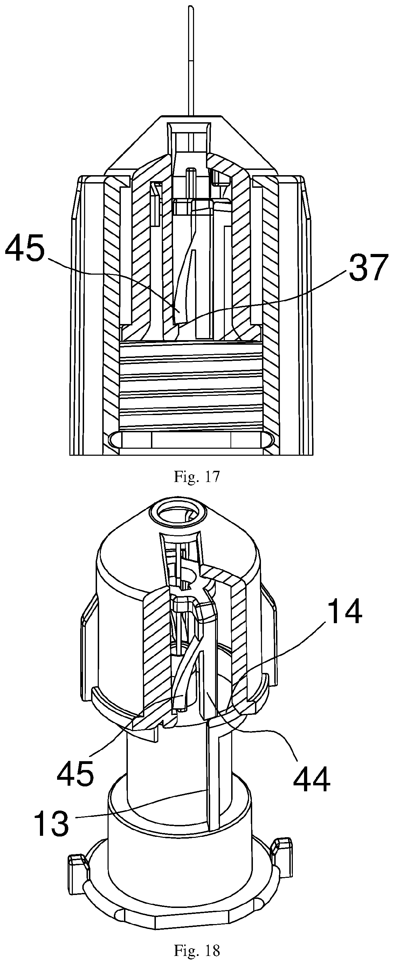

In the process of insulin injection, the shield axially moves, the spring is compressed, in the process of axial movement of the hooks of the second groove walls, as shown in FIG. 16, the hooks firstly move to make contact with outer sides of the elastic arms and then continue to axially move, each elastic arm is extruded towards one side of the corresponding support arm till, as shown in FIG. 17, upper surfaces of the hooks move to positions below the elastic arms, the elastic arms reset to the sides away from the support arms at the time and make contact with the second groove walls, lower ends of the elastic arms are located above the hooks, in the state, the elastic arms are still in an elastic energy storage state and the shield is in the retraction state.

After the injection is finished, the shield axially moves to cover the syringe needle under the reset action of the spring, in the process, the hooks act on the lower ends of the elastic arms to drive the support body to axially move till the lower ends of the support arms disengage from the first groove walls, stored elastic energy of the elastic arms is further released at the time to drive the support part to rotate to the lower ends of the support arms and above the support surfaces, the state is shown in FIG. 18, and the support part is supported between the top wall of the shield and the support surfaces of the needle hub.

In the state, since the maximum width formed by the elastic arms and the support arms is larger than the width of the sliding grooves, the elastic arms and the support arms cannot enter the sliding grooves again, so that the support part is made to be supported in a space between the shield and the needle hub, then, the shield is pressed again, and the needle tip cannot be exposed again based on a supporting function of the support part.

In the state, the sides, close to the first groove walls, of the support surfaces are required not to be lower than the sides away from the first groove walls. As a preferable scheme, each support surface is gradually lowered from one side of the corresponding first groove wall to the side away from the corresponding first groove wall, and accordingly, when the shield is pressed, the shield moves along a slope in a direction away from the sliding grooves, thereby further improving protection reliability.

In short, the above descriptions are merely the better embodiments of the present invention, which are not used for limiting the present invention. Any modification, equivalent replacement, improvement, etc. made within the spirit and principle of the present invention shall fall within the scope of protection of the present invention.

## Claims

1. An automatically-triggered needle protector, at least comprising:
a needle hub provided with a syringe needle, wherein at least one first groove wall downwards extending from an upper end of the needle hub is arranged on an outer side of the needle hub in a circumferential direction, and support surfaces extending from top ends of the first groove walls in a direction away from the first groove walls are arranged at the upper end of the needle hub;
a protective sleeve sleeved an outer side of the needle hub and fixedly connected to the needle hub, wherein a first cavity with an upper end open is formed between the protective sleeve and the needle hub;
a shield installed in the first cavity, wherein the shield has an extension state and a retraction state in the first cavity and is configured to be movable between the extension state and the retraction state, an inner side of the shield is provided with second groove walls opposite to the first groove walls, a sliding groove is formed between each first groove wall and the corresponding second groove wall, and a lower end of each second groove wall is provided with a hook extending towards one side of the corresponding first groove wall;
a spring installed in the first cavity and located between the shield and a cavity bottom of the first cavity; and
a support part, wherein the support part at least comprises a support base body and support arms corresponding to the sliding grooves, the sides, facing the second groove walls, of the support arms are provided with elastic arms, upper ends of the elastic arms are located at positions, close to upper ends, of the support arms, a first gap is arranged between a lower end of each elastic arm and the corresponding support arm, and a maximum width formed by the elastic arms and the support arms is larger than a width of the sliding grooves; and
the support part at least comprises an initial state and a support state and is configured to be switched into the support state from the initial state, wherein in the initial state, the support base body of the support part is in contact with the upper end of the needle hub; and in the support state, the support part disengages from the sliding grooves, and lower ends of the support arms are located above the support surfaces.

2. The automatically-triggered needle protector according to claim 1, **characterized in that** the two sliding grooves are evenly distributed in a circumferential direction.

3. The automatically-triggered needle protector according to claim 1, **characterized in that** a needle body installation portion is arranged at the upper end of the needle hub, the support base body of the support part is provided with an installation hole adaptive to the needle body installation portion, a circumferential limiting mechanism is arranged between the needle body installation portion and the support base body and comprises at least one limiting protrusion arranged on an outer side of the needle body installation portion and limiting grooves formed in an inner side of the installation hole and adaptive to the limiting protrusions.

4. The automatically-triggered needle protector according to claim 1, **characterized in that** the sides, close to the first groove walls, of the support surfaces are not lower than the sides away from the first groove walls.

5. The automatically-triggered needle protector according to claim 1, **characterized in that** a first axial guide mechanism and an axial anti-disengaging limiting mechanism are arranged between the shield and the protective sleeve.

6. The automatically-triggered needle protector according to claim 1, **characterized in that** the axial anti-disengaging limiting mechanism comprises a first limiting step arranged at an upper-end open position of the protective sleeve and a second limiting step arranged on an outer side of a lower end of the shield.

7. The automatically-triggered needle protector according to claim 1, **characterized in that** the upper end of the needle hub is provided with limiting convex portions with upper ends protruding out of the support surfaces, one side of each limiting convex portion is provided with a first limiting surface opposite to a corresponding first groove wall, and the other side of each limiting convex portions is provided with a second limiting surface connected to the end, away from the corresponding first groove wall, of the corresponding support surface.

8. An insulin pen needle, at least comprising the automatically-triggered needle protector according to any one of claims 1-7 and an initial protective cover an outer side of the automatically-triggered needle protector.

9. The insulin pen needle according to claim 8, **characterized in that** a second axial guide mechanism is arranged between the protective sleeve and the initial protective cover.

10. The insulin pen needle according to claim 8, **characterized in that** the shield is provided with a top wall, the top wall is provided with a needle via a hole for containing a syringe needle to pass through and further provided at least one through groove penetrating through the top wall, limiting rods corresponding to the through grooves and configured into an initial state are arranged in the initial protective cover, and the limiting rods penetrate through the through grooves and abut against the upper surface of the support base body.
